# FASCICULE DE BREVET EUROPEEN

(11) **EP 2 282 727 B1**
(45) Date de publication et mention de la délivrance du brevet: **23.11.2011**
(21) Numéro de dépôt: 09738359.0
(22) Date de dépôt: 28.04.2009
(51) Int. Cl.: A61K 9/51

(54) **MICELLES POLYMERISEES**
POLYMERISIERTE MIZELLEN
POLYMERIZED MICELLES

(30) Priorité: 29.04.2008 FR 0802390
(43) Date de publication de la demande: 16.02.2011
(73) Titulaire: Les Laboratoires Servier, 92284 Suresnes Cedex (FR); COMMISSARIAT A L'ENERGIE ATOMIQUE, 75015 Paris (FR)
(72) Inventeur: OGIER, Julien, 75015 Paris (FR); DORIS, Eric, F-91400 Orsay (FR); LEFOULON, François, F-45000 Orléans (FR); ARNAULD, Thomas, F-45000 Orléans (FR)
(74) Mandataire: Bestel, Delphine
(86) Numéro de dépôt international: PCT/FR2009/000493
(87) Numéro de publication internationale: WO 2009/133325

(56) Documents cités:
- US-A1- 2002 064 513
- GOTO ET AL.: "Micellar Behaviour of Sugar-Carrying Polystyrene in Aqueous Solution" MACROMOL.CHEM. PHYS., vol. 202, no. 7, 2001, pages 1161-1165, XP002505668 Weinheim

## Description

L'invention a pour objet des micelles polymérisées, leurs procédés de préparation et leurs applications.

Un grand nombre de molécules à activité thérapeutique et en particulier les molécules anti-cancéreuses présentent une faible solubilité dans l'eau, provenant des propriétés intrinsèques hydrophobes de la molécule. En effet, ces molécules doivent avoir un certain degré d'hydrophobicité pour pouvoir être internalisées dans les cellules. La vectorisation des molécules thérapeutiques a pour but de contourner les problèmes associés en particulier à la solubilité, la stabilité, la pharmacocinétique, la biodistribution desdites molécules et leur ciblage spécifique.

Différents systèmes de vectorisation de composés hydrophobes ont été décrits dans la littérature comme pouvant transporter des quantités suffisantes de molécules à activité thérapeutique à travers les différentes barrières biologiques pour atteindre efficacement leur site d'action.

Une de ces techniques de vectorisation consiste à encapsuler un principe actif dans une microsphère formée par une matrice de polymères tels que les poly(alkylcyanoacrylates), les poly(anhydrides), l'acide poly(lactique). Ces microsphères ont des tailles de 20µm à 100µm excluant leur usage par voie intra-veineuse et permettent des taux d'inclusion relativement faible entre 0.2% et 3.5% de composés hydrophobes.

Les micelles de polymères, autre technique de vectorisation, désignent des dispersions colloïdales composées de polymères amphiphiles avec des domaines hydrophiles et hydrophobes distincts. Ces micelles de polymères sont des structures supramoléculaires en coeur/coque. Les micelles de polymères sont composées de polyéthers utilisés en combinaison avec des poly(éthylène glycol) pour former des polymères amphiphiles sous forme de copolymères diblocs (pluroniques : PPO-co-PEG) ou de copolymères triblocs (poloxamers : PEG-co-PPO-co-PEG). Ces micelles de polymères ont une taille comprise entre 50nm et 100nm. Les taux d'inclusion de composés dans les micelles de polymères sont compris entre 0. 1 % et 40% selon la méthode d'inclusion utilisée : inclusion par évaporation, inclusion par dialyse ou inclusion par nanoprécipitation. Les micelles de polymères sont donc des vecteurs permettant l'incorporation de quantités importantes de composés hydrophobes mais nécessitant une mise en oeuvre (synthèse et inclusion) technique et industrielle difficile.

Les systèmes de vectorisation par les liposomes et les vésicules de polymères sont étudiés de façon intensive et plusieurs formulations de médicaments par ces systèmes sont actuellement en phase clinique dont certaines ont même été approuvées pour leur utilisation clinique. Les liposomes et les vésicules de polymères peuvent inclure des principes actifs hydrophiles dans le coeur aqueux de la vésicule ou des molécules hydrophobes dans la bicouche de polymères. Les liposomes et les vésicules de polymères ont des structures (MLV vésicules multilamellaires, SUV vésicules unilamellaires de petites tailles, LUV vésicules unilamellaires de grande taille, GUV vésicules géantes) et des tailles très différentes comprises entre 100nm et 1000nm. Les liposomes et les vésicules de polymères sont des véhicules de principes actifs importants dans la vectorisation de molécules hydrophiles.

En 2003, un nouveau type d'organisation à la surface des nanotubes de carbone a été mise en évidence : les nanobagues, i.e. une structuration d'amphiphiles en anneaux sur toute la longueur des nanotubes. Les nanobagues sont des anneaux de surfactants polymérisés formés à la surface des nanotubes puis séparés de leur support carboné pour les utiliser comme agents de solubilisation de principes actifs hydrophobes

(WO 2004/092231). Les nanobagues sont cependant des nanovecteurs difficilement industrialisables.

Les vecteurs de délivrance décrits supra répondent plus ou moins bien au challenge de la vectorisation qui est de transporter suffisamment de principe actif de façon efficace et peu toxique afin de traiter la pathologie. Ces vecteurs répondent au problème de la solubilité des principes actifs hydrophobes sans pour autant résoudre les problèmes de taille de vecteur, de mise en oeuvre ou d'industrialisation. La présente invention a donc pour but de proposer une nouvelle stratégie pour obtenir des nanovecteurs ayant une capacité d'inclusion des principes actifs hydrophobes supérieure à celle des vecteurs classiques de la littérature et ayant une formulation simple facilitant son industrialisation future.

La présente invention concerne des micelles polymérisées caractérisées en ce qu'elles comprennent des molécules amphiphiles polymérisées obtenues à partir de molécules amphiphiles comportant une ou deux chaînes lipidiques comprenant chacune un ou deux motifs polymérisables, de types diacétylénique, vinyle, acrylate ou styrène, liées à une tête polaire.

Selon la présente invention, on entend par « micelles » des objets sphériques auto-assemblés avec une surface hydrophile et un coeur lipophile et dont la taille est inférieure à 100nm.

On entend par « amphiphiles » ou « surfactants » des molécules organiques ayant la particularité d'être à la fois hydrophile et hydrophobe. Les amphiphiles, caractérisés par leurs propriétés antagonistes, ont des propriétés particulières en solution et s'organisent spontanément en milieux aqueux sous différentes microstructures.

L'invention porte sur des micelles polymérisées comprenant des molécules amphiphiles polymérisées obtenues à partir de molécules amphiphiles de formule générale A-X-B-L-Z où A représente CH₃―(CH₂ 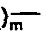 C≡C―C≡C―(CH₂ 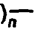 ou CH₂=CH- ou CH₂=CH-C₆H₄- , n et m, identiques ou différents, étant des entiers de 1 à 16; où X représente CO-NH ou NH-CO ou une liaison, X est une liaison si B est une liaison et L est une liaison ; où B représente ―(CH₂ 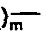 C≡C―C≡C―(CH₂ 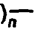 ou -CH=CH-C₆H₄- ou une liaison, n et m, identiques ou différents, étant des entiers de 1 à 16 ; où L représente -(CH₂)ᵣ-CH[NH-CO-A']- ou une liaison, r étant un entier de 1 à 16 et A' représente A ;
où Z représente 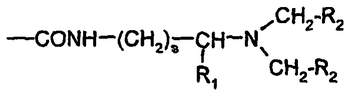 ou 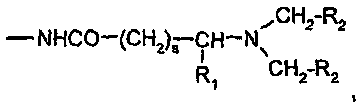 s étant un entier de 1 à 16 avec R₂ représentant COOH ou SO₃H ou OSO₃H ou OPO₃H₂ ou OPO₂H₂ avec R₁ représentant H ou un radical COOH ou SO₃H ou OSO₃H ou OPO₃H₂ ou OPO₂H₂ ou un groupe -CO-NH-(CH₂)ₜ-CH₃, t étant un entier de 1 à 16, ou Z peut être également des têtes polaires hydrophiles neutres, de type sucre ou polysaccharide, ainsi que leurs sels d'addition avec un acide ou une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesuifonique, camphorique.

Parmi les bases pharmaceutiquement acceptables, on peut citer à titre non limitatif l'hydroxyde de sodium, l'hydroxyde de potassium, la triéthylamine, la tertbutylamine.

De préférence, l'invention concerne des micelles polymérisées comprenant des molécules amphiphiles polymérisées obtenues à partir de molécules amphiphiles de formule générale A-X-B-L Z
où A représente 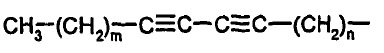 n et m, identiques ou différents, étant des entiers de 1 à 16;
où X représente CO-NH ou NH-CO ou une liaison, X est une liaison si B est une liaison et L est une liaison;
où B représente 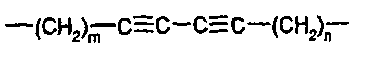 ou une liaison, n et m, identiques ou différents, étant des entiers de 1 à 16 ;
où L représente -(CH₂)ᵣ-CH[NH-CO-A']- ou une liaison, r étant un entier de 1 à 16 et A' représente A ou CH₂=CH- ou CH₂=CH-C₆H₄- ;
où Z représente 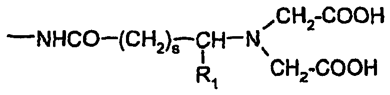 ou 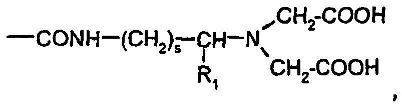 s étant un entier de 1 à 16 avec R₁ représentant H ou un radical COOH ou un groupe -CO-NH-(CH₂)ₜ-CH₃, t étant un entier de 1 à 16, ou Z peut être également des têtes polaires hydrophiles neutres, de type sucre ou polysaccharide, ainsi que leurs sels d'addition avec un acide ou une base pharmaceutiquement acceptable.

L'invention s'étend également aux micelles polymérisées constituées de molécules amphiphiles dont les têtes polaires hydrophiles neutres sont de type éthers couronnes.

Avantageusement, l'invention porte sur des micelles polymérisées dont la tête polaire Z est fonctionnalisée.

Par micelles polymérisées « fonctionnalisées », on entend des micelles polymérisées modifiées par des ligands à reconnaissance moléculaire pour les faire évoluer vers des vecteurs intelligents et sélectifs afin d'identifier de façon spécifique des antigènes ou des récepteurs surexprimés à la surface de cellules cibles telles que les cellules cancéreuses ou infectieuses. Pour cela, les micelles polymérisées doivent être fonctionnalisées par des ligands spécifiques dont l'accroche à la surface du vecteur se fait par un couplage chimique.

Avantageusement, les ligands accrochés à la surface des micelles polymérisées sont : les fluorophores ou agents d'imagerie nucléaire (⁹⁹Tc, ¹¹In, ¹²⁵I ¹⁸F, ⁶⁴Cu) ou optique (cyanine, fluorescéine, luciférase, quantum dots) ou magnétique (particules d'oxyde de fer), l'acide folique, le mannose, le galactose, des anticorps, des ligands de type RGD.

De manière préférée, les micelles polymérisées selon l'invention sont fonctionnalisées au niveau de la tête polaire Z par un acide folique.

Dans un mode de réalisation préféré, les micelles polymérisées sont fonctionnalisées selon un procédé tel que décrit dans l'exemple 5.

La fonctionnalisation de surface des micelles polymérisées rend donc lesdites micelles polymérisées furtives et permet le ciblage de cellules spécifiques.

L'invention s'étend également aux micelles polymérisées incluant un ou des composés hydrophobes au sein des amphiphiles polymérisés selon l'invention.

On entend par « composés hydrophobes » des molécules de petites taille possédant une faible solubilité dans l'eau inférieure à 1g par litre dans tout ou partie de la zone de pH ou des protéines ou des acides nucléiques ayant des problèmes de solubilité ou de stabilité en milieux aqueux.

De manière préférée, les micelles polymérisées selon l'invention sont constituées des molécules amphiphiles polymérisées obtenues à partir de la molécule amphiphile II-4 de formule

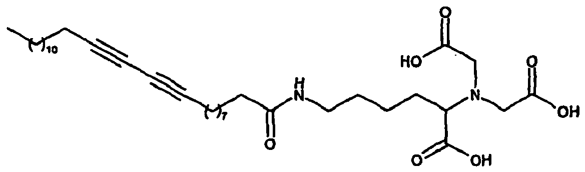

De manière également préférée, les micelles polymérisées sont constituées des molécules amphiphiles polymérisées obtenues à partir de la molécule amphiphile II-23 de formule

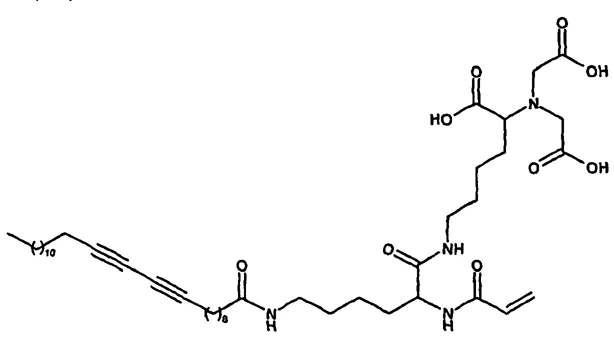

Avantageusement, les micelles polymérisées selon l'invention sont constituées des molécules amphiphiles polymérisées obtenues à partir de la molécule amphiphile de formule

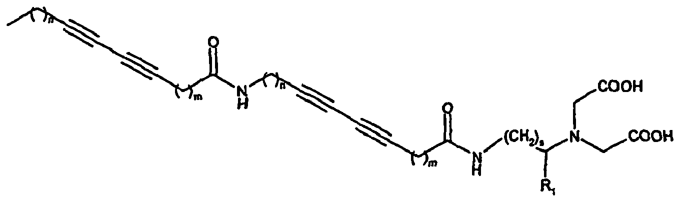

Les micelles polymérisées sont également formées à partir des molécules amphiphiles polymérisées obtenues à partir de la molécule amphiphile de formule

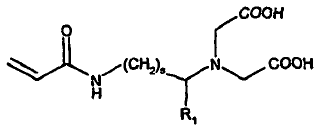

De manière préférée, les micelles polymérisées selon l'invention sont constituées des molécules amphiphiles polymérisées obtenues à partir de la molécule amphiphile de formule

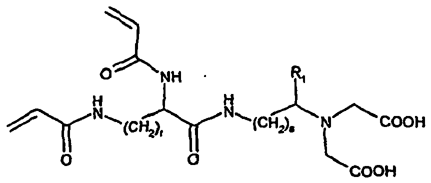

Préférentiellement, les micelles polymérisées sont formées à partir des molécules amphiphiles polymérisées obtenues à partir de la molécule amphiphile de formule

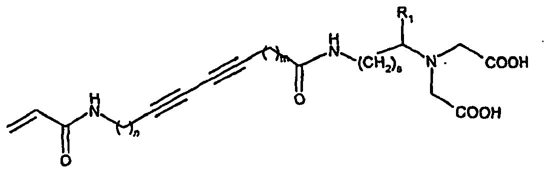

Avantageusement, les micelles polymérisées selon l'invention sont constituées des molécules amphiphiles polymérisées obtenues à partir de la molécule amphiphile de formule

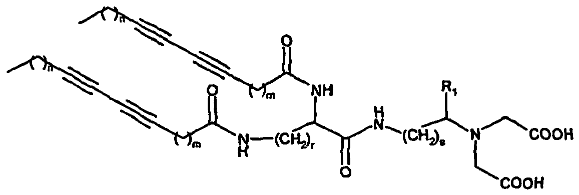

La présente invention a également pour objet le procédé de préparations des composés selon l'invention caractérisé en ce que :

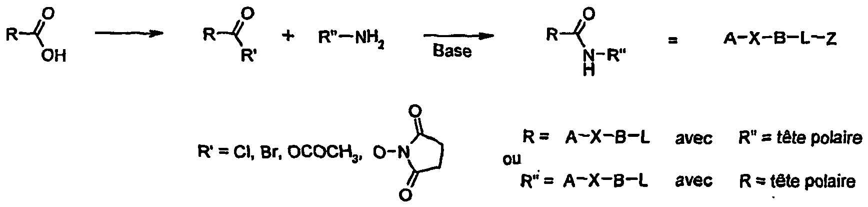

L'invention s'étend également aux compositions pharmaceutiques comprenant des micelles polymérisées selon l'invention. Avantageusement, ces compositions pharmaceutiques comprennent un ou plusieurs excipients pharmaceutiquement acceptables, i.e. un ou plusieurs véhicules ou excipients inertes, non toxiques et appropriés.

En ce qui concerne les excipients pharmaceutiquement acceptables, on peut citer à titre non limitatif les liants, les diluants, les délitants, les stabilisants, les conservateurs, les lubrifiants, les odorants, les aromatisants ou les édulcorants.

Parmi les compositions pharmaceutiques selon l'invention, on retiendra plus particulièrement celles qui conviennent pour l'administration par voie orale, parentérale et notamment intraveineuse, per ou transcutanée, nasale, rectale, perlinguale, oculaire, respiratoire et plus spécifiquement les comprimés simples ou dragéifiés, les comprimés sublinguaux, les gélules, les glossettes, les capsules, les tablettes, les préparations injectables, les aérosols, les gouttes oculaires ou nasales, les suppositoires, les crèmes, pommades ou gels dermiques.

La voie d'administration préférée est la voie intraveineuse et les compositions pharmaceutiques correspondantes peuvent permettre la libération instantanée ou différée des principes actifs.

La présente invention porte également sur l'utilisation des micelles polymérisées selon l'invention comme vecteurs de molécules hydrophobes. L'invention s'étend également à l'utilisation des micelles polymérisées comme vecteurs de principes actifs hydrophobes.

Par « vecteur », on entend toute molécule ou assemblage de molécules pouvant transporter un principe actif, une protéine ou un acide nucléique vers son site d'action par ciblage de ligands spécifiques ou de façon passive par solubilisation ou stabilisation de l'espèce en question

On entend par « principe actif » toute molécule, protéine ou acide nucléique possédant un effet thérapeutique.

Différentes méthodes d'inclusion de principes actifs sont développées afin d'optimiser le taux d'inclusion ou d'incorporation dans les micelles polymérisées. Les méthodes d'inclusion selon l'invention sont :
- la méthode « vortex » qui consiste en l'inclusion par agitation vortex d'une solution aqueuse de micelles polymérisées et de principe actif à l'état de poudre pendant environ 30 minutes ;
- la méthode « vortex/sonication » qui consiste en l'inclusion par agitation vortex combinées à des cycles de sonication d'une solution aqueuse de micelles polymérisées et de principe actif à l'état de poudre pendant environ 2 heures ;
- la méthode « agitation à 20°C » qui consiste en l'inclusion par agitation magnétique à température ambiante pendant environ 12 heures d'une solution aqueuse de micelles polymérisées et de principe actif à l'état de poudre ;
- la méthode « agitation à 50°C » qui consiste en l'inclusion par agitation magnétique à environ 50°C pendant environ 12 heures d'une solution aqueuse de micelles polymérisées et de principe actif à l'état de poudre ;
- la méthode « évaporation de DCM » qui consiste en l'inclusion par addition du principe actif solubilisé dans du dichlorométhane (DCM) à une solution aqueuse de micelles polymérisées chauffée à environ
50°C.

A l'issue de la séquence d'inclusion, la solution est filtrée afin d'éliminer l'excès de principe actif non inclus.

L'invention concerne, de manière préférée, une méthode d'inclusion par agitation magnétique pendant 12 heures à 50°C d'une solution de micelles polymérisées et de principe actif. L'inclusion de principe actif dans les micelles polymérisées est contrôlée par l'énergie d'agitation et la température. Le taux d'incorporation est amélioré par une agitation plus énergique, une mise en contact plus longue et une température plus élevée.

De préférence, les solutions micellaires obtenues à l'issue de l'étape d'inclusion sont stérilisées par filtration sur filtre de 0.22µm en vue d'injection en intraveineuse sans perte de concentration de principe actif.

L'étude d'inclusion menée sur les micelles a mis en évidence l'effet de la polymérisation de la micelle sur la capacité d'incorporation de principe actif. Les micelles doivent être polymérisées afin que ce nanovecteur ait la capacité d'inclure une quantité très importante de principes actifs. Un taux d'incorporation de S39625 de 48 % est obtenu pour ce transporteur, soit un taux 5 fois supérieur à celui obtenu avec la formulation nanobague.

L'étude d'inclusion menée sur différents principes actifs a confirmé l'aptitude ce de nanovecteur à solubiliser différents types de molécules hydrophobes avec de forts taux d'inclusion. Les travaux réalisés sur une molécule de référence, le paclitaxel, a permis de comparer les valeurs des taux d'incorporation avec les données de la littérature. Les micelles polymérisées de l'amphiphile II-4 ont permis d'inclure le paclitaxel à un taux d'inclusion de 33 %. Dans la littérature, des taux d'inclusion du paclitaxel compris entre 6,7 % et 14,3 % ont été atteints avec différents types de vésicules, des taux d'incorporation compris entre 0,2 % et 27 % avec des nanoparticules, et des taux d'inclusion compris entre 0,2 et 25 % avec les micelles de polymères. Les micelles polymérisées se présentent donc comme un nanovecteur avec un pouvoir de solubilisation remarquable qui a permis de solubiliser des principes actifs de structures et de poids moléculaires différents.

La présente invention porte enfin sur un procédé d'obtention des micelles polymérisées selon l'invention. Ce procédé d'obtention comprend les étapes suivantes :
- les composés lipidiques à polymériser selon l'invention sont auto-assemblés en micelles sphériques ;
- les micelles sphériques auto-assemblées sont polymérisées.

Par « auto-assemblage » de molécules amphiphiles, on entend l'organisation spontanée en micelles sphériques des molécules amphiphiles en milieux aqueux à une concentration supérieure à la concentration micellaire critique ou CMC.

La concentration micellaire critique de la molécule amphiphile II-4 telle que décrite supra a été déterminée expérimentalement à 0.082mg/ml. L'invention s'étend également au procédé d'obtention de micelles polymérisées dans lequel l'étape de polymérisation est de type irradiation lumineuse ou photopolymérisation.

La photopolymérisation est une méthode de polymérisation particulièrement bien adaptée à la polymérisation des motifs diacétyléniques, La photopolymérisation est une méthode « propre » utilisant une irradiation lumineuse à 254nm et aucun agent chimique externe. La photopolymérisation des motifs diacétyléniques implique la formation d'intermédiaires diradicalaires : la première étape consiste à former l'espèce diradicale par une excitation photonique ; la deuxième étape est la réaction de propagation du radical à un nouveau motif polymérisable se trouvant à proximité, faisant ainsi grandir la chaîne polymérique ; la dernière étape est une étape de terminaison par couplage de deux radicaux.

L'invention s'étend aussi au procédé d'obtention de micelles polymérisées dans lequel l'étape de polymérisation est de type polymérisation radicalaire. Les motifs vinyles et acrylates sont généralement polymérisés par polymérisation radicalaire. Cette voie de polymérisation est connue et couramment utilisée. L'initiation de la polymérisation radicalaire peut-être effectuée à l'aide d'un initiateur radicalaire généré par dissociation thermique et homolytique, réaction d'oxydo-réduction ou par des irradiations.

Enfin, l'invention s'étend également au procédé d'obtention de micelles polymérisées dans lequel l'étape de polymérisation comprend plusieurs types de polymérisation successives, par exemple une photopolymérisation puis une polymérisation par initiateur radicalaire.

La présente invention est illustrée sans pour autant être limitée par les figures et exemples suivants :

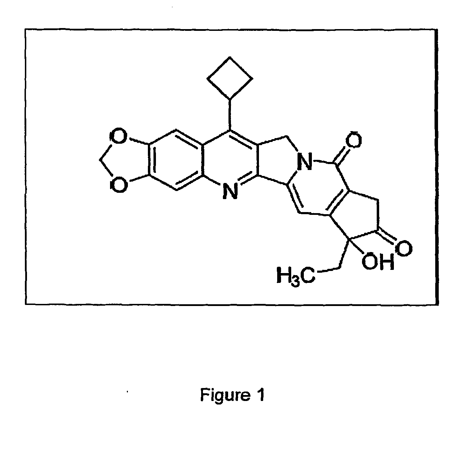

Figure 1 : Structure chimique du S39625 ;
Figure 2: Méthode d'inclusion du S39625 dans les micelles polymérisées et non-polymérisées ;
Figure 3 : (1) chromatogramme du S39625 dans l'acétonitrile, (2) chromatogramme des micelles non-polymérisées / polymérisées, (3) chromatogramme du S39625 inclus dans les micelles non-polymérisées, (4) chromatogramme du S39625 inclus dans les micelles polymérisées ;

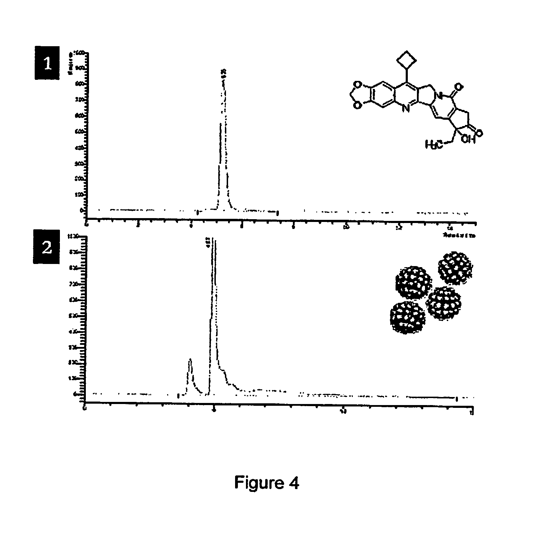

Figure 4: (1) chromatogramme du S39625 solubilisé dans l'acétonitrile, (2) chromatogramme du S39625 inclus dans les micelles polymérisées ;
Figure 5: Structures des principes actifs hydrophobes (1) S44563, (2) 42909, (3) paclitaxel et (4) pyrène ;
Figure 6: Structure chimique du 4-amino-3-hydroxy-1-sulfonatenaphthalène ;
Figure 7: Spectres de fluorescence du 4-amino-3-hydroxy-1-sulfonatenaphthalène, des nanobagues et des micelles polymérisées fonctionnalisées par le fluorophore et de l'échantillon de contrôle (excitation à 345nm) ;
Figure 8: (A) spectres d'absorbance de l'amine III-9, des micelles polymérisées et des micelles polymérisées fonctionnalisées par l'amine III-9 (B) spectres de fluorescence de l'amine III-9, des micelles polymérisées fonctionnalisées par l'amine III-9 et de l'échantillon de contrôle (excitation à 280nm).

### EXEMPLE 1 : SYNTHESE DES MOLECULES AMPHIPHILES II-4 ET II-23

### 1.1 Synthèse de la molécule amphiphile 11-4

La première étape de cette synthèse selon le schéma 1 consiste à préparer la partie hydrophile de la molécule amphiphile (dérivé de l'acide nitrilotriacétique) à partir de la N-benzyloxycarbonyl-L-lysine (Z-L-lysine).

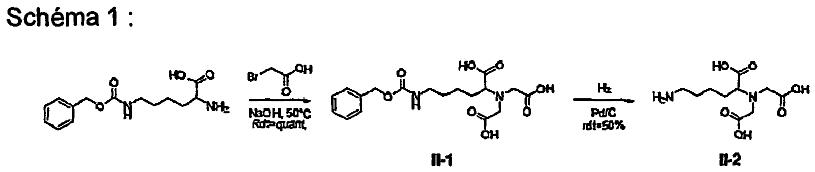

La deuxième étape de la synthèse selon le schéma 2 consiste à coupler la tête hydrophile II-2 avec l'acide 10,12-pentacosadiynoique préalablement activé.

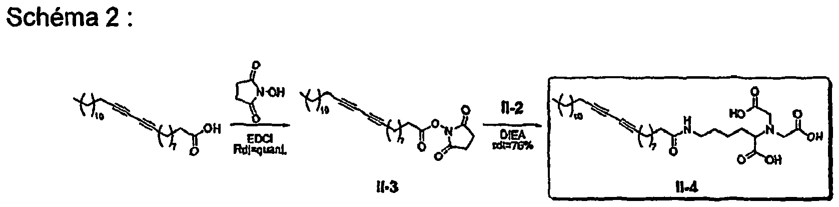

L'acide pentacosadiynoique est activé par le N-hydroxysuccinimide en présence de N-(3-dimethylaminopropyl)-N'-ethylcarbodiimide pour donner l'acide activé II-3. Le tensio-actif II-4 est obtenu par le couplage peptidique entre la tête hydrophile NTA II-2 et de l'acide activé II-3. Le produit pur est obtenu par précipitation dans l'eau par ajout d'acide chlorhydrique.

### 1.2 Synthèse de la molécule amphiphile II-23

La molécule amphiphile II-23 est un surfactant comportant deux types de motifs polymérisables : un motif diacétylénique polymérisable par irradiation lumineuse à 254nm et un motif acrylate réticulable par bombardement électronique.

La première étape de la synthèse consiste à préparer la partie hydrophile de la molécule amphiphile (tête NTA) à partir de la N-benzyloxycarbonyl-L-lysine (Z-L-lysine) conformément au schéma 1 puis de protéger les acides carboxyliques sous forme d'esters ter-butyliques. On obtient la tête hydrophile protégée tBu-NTA nommée II-14.

La deuxième étape de la synthèse est résumée dans le schéma réactionnel 3 suivant.

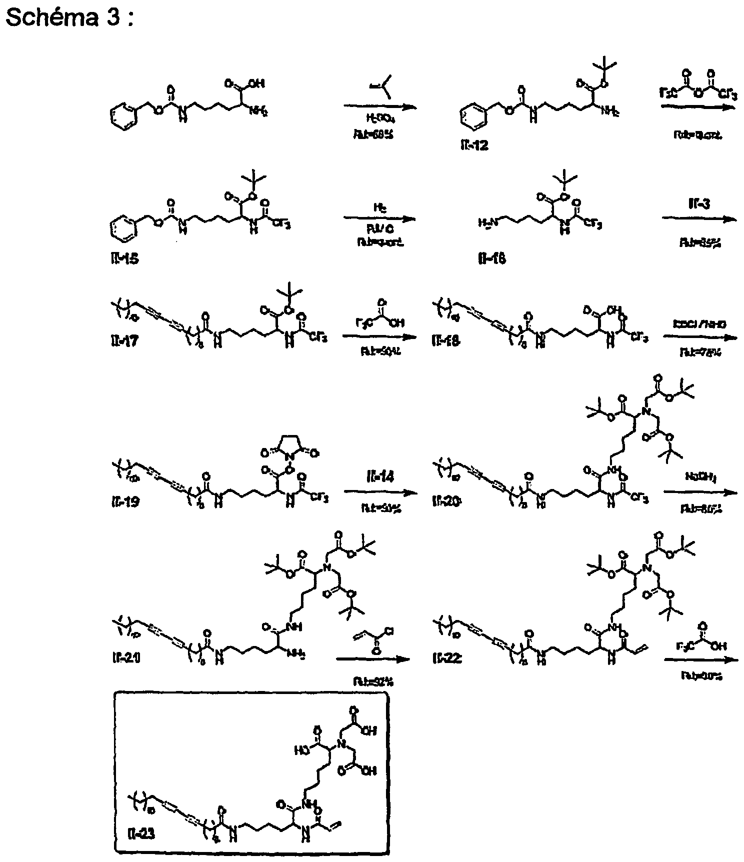

### EXEMPLE 2 : PREPARATION ET CARACTERISATION DES MICELLES POLYMERISEES

### 2.1 Préparation des micelles polymérisées

Les micelles sphériques sont des arrangements d'amphiphiles obtenus à partir de la concentration micellaire critique (CMC). Ces organisations de forme sphérique ont une surface hydrophile et un coeur lipophile ou hydrophobe.

Le tensio-actif II-4 s'arrange sous forme de micelles sphériques lorsque le pH de la solution aqueuse est supérieur à 10. Au delà du pH=10, le doublet non liant de l'amine tertiaire de la partie hydrophile du surfactant est libéré conduisant à l'augmentation du volume de la tête polaire, et en dessous de cette valeur de pH des liaisons hydrogènes peuvent s'établir entre les carboxylates et l'hydrogène de l'amine tertiaire protonée conduisant à la diminution du volume de la tête NTA de l'amphiphile II-4.

Les micelles auto-assemblées et polymérisées d'amphiphile II-4 sont préparées selon le schéma 4 par irradiation lumineuse à 254nm d'une solution aqueuse à pH=12 de II-4 pendant 5 heures puis le pH est ajusté au pH physiologique par dialyse contre une solution à un pH compris entre 7 et 8.

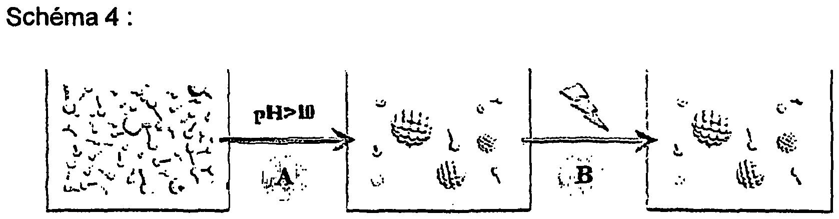

On observe que la polymérisation par irradiation lumineuse de l'amphiphile II-4 structuré sous forme de micelles est un processus relativement lent et qu'une conversion de la totalité des amphiphiles II-4 en polydiacétylénique devrait être atteinte pour des temps de polymérisation au-delà de 5 heures.

### 2.2 Caractérisation des micelles polymérisées

L'analyse des micelles polymérisées d'amphiphiles II-4 est réalisée par granulométrie laser. Cette technique, basée sur la diffusion quasi élastique de la lumière et la spectroscopie croisée de corrélation de photon, permet de déterminer la distribution en taille d'un échantillon.

L'analyse en intensité d'un échantillon de micelles polymérisées à 50mg/ml par l'appareil Nanophox présente un pic principal de population à 4,9nm. L'analyse en nombre de l'échantillon de micelles polymérisées à 1mg/ml par l'appareil Malvem Zetasizer a mis en évidence la présence d'une population unique à 5,1nm.

La polymérisation des micelles induit un phénomène de contraction des micelles pouvant s'expliquer par le fait que la polymérisation fige l'assemblage des amphiphiles et limite les phénomènes de gonflements.

### EXEMPLE 3 : INCLUSION DU S39625 DANS LES MICELLES POLYMERISEES ET DANS LES MICELLES NON-POLYMERISEES

### 3.1 Méthode d'inclusion du S39625

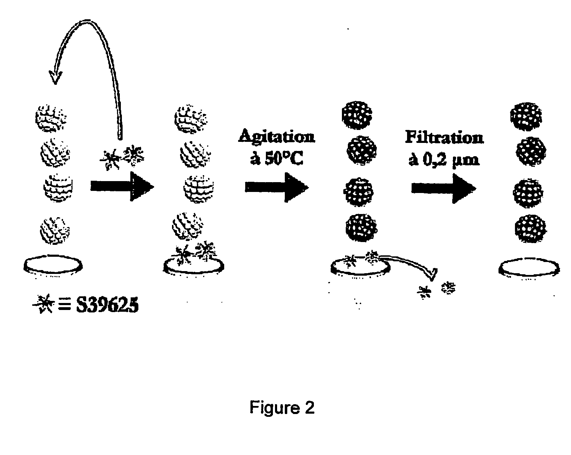

Le S39625 a été encapsulé dans une solution de 10mg/ml de micelles non-polymérisées et de micelles polymérisées par la méthode « d'agitation et chauffage à 50°C » telle que détaillée dans la description et représentée Figure 2.

### 3.2 Détermination du taux d'inclusion du S39625 par HPLC

Le taux d'inclusion du S39625 est déterminé par la méthode HPLC en phase inverse. Cette technique se base sur la différence d'interaction des molécules entre la phase mobile et la phase stationnaire. Cette différence se répercute sur les temps de rétention de ces molécules. Pour des conditions HPLC données, une même molécule a toujours le même temps de rétention. Les conditions HPLC en phase inverse pour la détermination du taux d'inclusion du S39625 sont une colonne de silice greffée RP-18^{c} pour la phase stationnaire et un gradient d'eau et d'acétonitrile de 5% à 100% pour la phase mobile. La détection du S39625 en sortie de colonne est réalisée par un détecteur UV à une longueur d'onde de 385nm. Une courbe d'étalonnage du S39625 dans l'acétonitrile a été réalisée par HPLC inverse. Dans ces conditions d'HPLC, le principe actif S39625 présente un temps de rétention de 25,8 minutes.

Le temps de rétention du S39625 encapsulé dans des micelles polymérisées a également été déterminé par HPLC en phase inverse pour laquelle le gradient de solvant est remplacé par un régime isocratique en acétonitrile pur. L'acétonitrile est un des meilleurs solvants du S39625 et son utilisation en tant qu'éluant permet de s'assurer du relargage très rapide du principe actif et de son élution comme une entité libre.

### 3.3 Résultats du taux d'inclusion du S39625

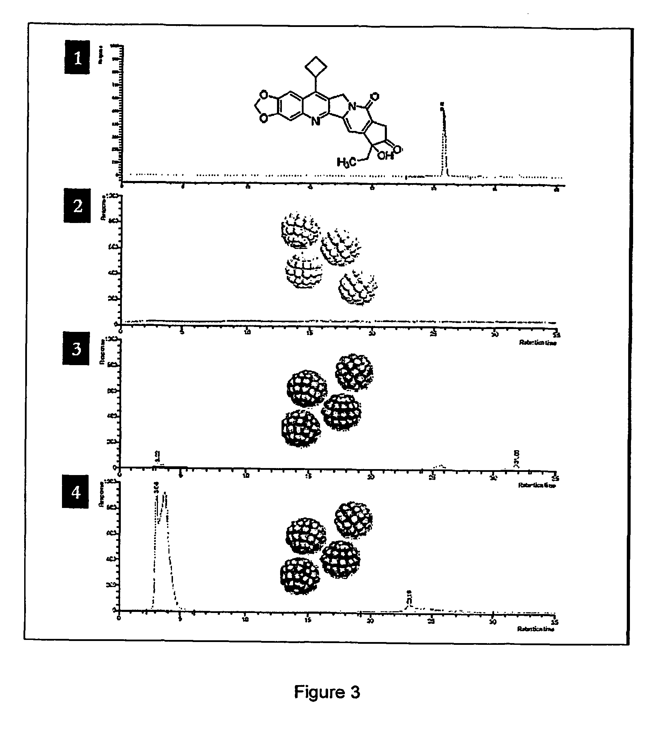

Les résultats d'incorporation du S39625 sont calculés à partir de la Figure 3.

Le chromatogramme du S39625 inclus dans les micelles non-polymérisées présente, le trois pics à 3,2 minutes, 25,8 minutes et 31,9 minutes. Au vu de l'aire du pic à 25,8 minutes, l'incorporation du principe actif dans les micelles non-polymérisées est compris entre 1,7 et 3,3%.

Le chromatogramme du S39625 inclus dans les micelles polymérisées présente un large pic à un temps de rétention très faible. Cette forte diminution du temps de rétention montre d'une part, que l'hydrophobicité du S39625 est fortement masquée par la surface très hydrophile de la micelle polymérisée et d'autre part que l'association S39625/micelles polymérisées est particulièrement robuste.

Le chromatogramme du S39625 solubilisé dans l'acétonitrile présente un pic à 5,2 minutes alors que le S39625 inclus dans la micelle polymérisée présente deux pics à 4,0 et à 4,9 minutes. Le taux d'inclusion dans les micelles polymérisées du S39625 et de ses produits de dégradation est estimé par intégration des deux pics. Le taux d'incorporation de ces molécules dans les micelles polymérisées est donc de 48%. Pour une concentration en amphiphile identique (10 mg/ml), la solubilité et donc la capacité d'encapsulation des micelles polymérisées est améliorée d'un facteur 5 par rapport aux nanobagues et d'un facteur 13 par rapport aux micelles non polymérisées.

### EXEMPLE 4 : INCLUSION DE PRINCIPES ACTIFS HYDROPHOBES DANS LES MICELLES POLYMERISEES

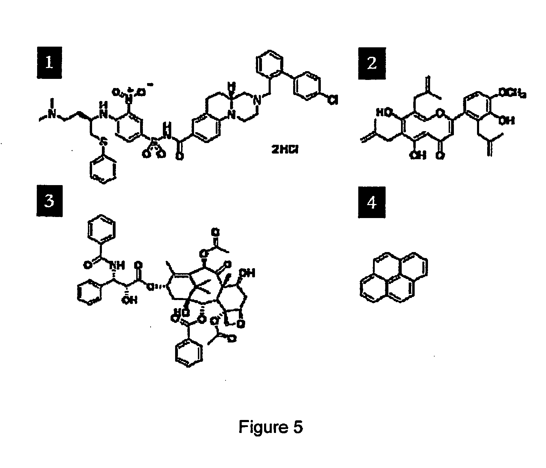

Des tests d'inclusion sont menés sur des principes actifs hydrophobes S42909, S44563, le paclitaxel une molécule active hydrophobe de référence et le pyrène une molécule très hydrophobe. Les formules chimiques de ces principes actifs hydrophobes sont représentées Figure 5. L'inclusion de ces principes actifs a été menée avec des solutions de micelles polymérisées à 10mg/ml par la méthode de chauffage à 50°C pendant 12 heures.

L'ensemble des résultats du Tableau 1 présente une forte incorporation des molécules hydrophobes dans les micelles polymérisées avec des taux d'inclusion supérieurs à 24% et une augmentation de la solubilité des principes actifs dans l'eau de 30 000 à 500 000.

**Tableau 1 :**

| Principe Actif Molécule | Masse molaire (g/mol) | Solubilité dans l'eau de la la molécule (en µg/mL) | Taux d'inclusion massique^{f} (en %) | Taux d'inclusion molaire⁹ (en %) | Nombre de molécules par micelle^{h} | Solubilité dans l'eau (en mg/mL) |
|---|---|---|---|---|---|---|
| S 39625 | 430 | 0,09 | 48 | 56 | 125 | 9,00 |
| S44563 | 912 | 2,00 | 45 | 36 | 56 | 8,30 |
| S42909 | 462 | 0,20 | 24 | 29 | 42 | 3,15 |
| Paclitaxel | 853 | 0,40 | 31 | 25 | 33 | 4,55 |
| Pyrène | 202 | 0,01 | 34 | 63 | 167 | 5,25 |

| | | | | | | |
|---|---|---|---|---|---|---|
| ^{f} Ratio massique (en %) entre médicament et médicament + transporteur x 100 dans la formulation après traitement ^{g} Ratio molaire (en % entre médicament et médicament + Amphiphile II-4 ^{h} x 100 dans la formulation après traitement Ratio molaire entre médicament et Amphiphile II-4 x nombre d'amphiphile/micelle (100 molécules) | | | | | | |

### EXEMPLE 5 : FONCTIONNALISATION DES MICELLES POLYMERISEES

### 5.1 Fonctionnalisation par le 4-amino-3-hydroxy-sulfonatenaphthalène

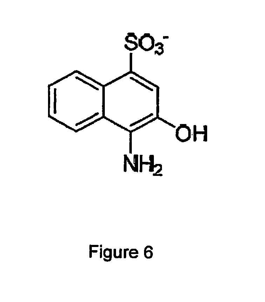

Les micelles polymérisées sont fonctionnalisées par un fluorophore hydrophile, le 4-amino-3-hydroxy-sulfonatenaphthalène décrit Figure 6. Ce fluorophore a une longueur d'onde d'excitation de 340nm et une longueur d'onde d'émission de 455nm.

L'accroche du fluorophore s'effectue par un couplage peptidique dans l'eau à pH basique (pH=12) avec comme agent de couplage le dicyclohexylcarbodiimide (DCC). Ce greffage a été réalisé en fort excès de fluorophore (50 équivalents) et d'agent de couplage (100 équivalents), et les échantillons ont été purifiés par colonne d'exclusion de taille.

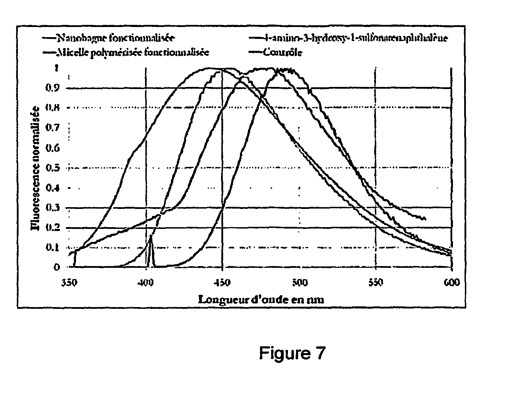

La fonctionnalisation des micelles polymérisées a été suivie par spectroscopie à fluorescence afin de mettre en évidence le greffage du fluorophore à la surface du nanovecteur. Les spectres de fluorescence des micelles polymérisées fonctionnalisées montrent une évolution du pic de fluorescence du 4-amino-3-hydroxy-sulfonatenaphthalène avec un shift bathochrome de fluorescence de 492nm (Figure 7). Le contrôle met en évidence que le shift de fluorescence des micelles polymérisées provient bien du greffage covalent du fluorophore à la surface du nanovecteur.

### 5.2 Fonctionnalisation par de l'acide folique

Le récepteur folate étant surexprimé à la surface des cellules cancéreuses, l'acide folique est très largement utilisé pour le ciblage spécifique des cellules cancéreuses du cerveau, du rein, du sein, des ovaires et des poumons.

Pour réaliser l'étape de couplage de l'acide folique aux micelles polymérisées, l'acide folique a été modifié afin d'introduire une fonction amine permettant le greffage. Le couplage du dérivé aminé de l'acide folique III-9 à la surface des micelles polymérisées s'effectue par un couplage peptidique dans l'eau à un pH basique (pH=12) avec de la DCC comme agent de couplage. Ce greffage est réalisé en fort excès de dérivé aminé de l'acide folique (50 équivalents) et d'agent de couplage (100 équivalents). L'échantillon a été purifié par filtration puis par colonne d'exclusion de taille.

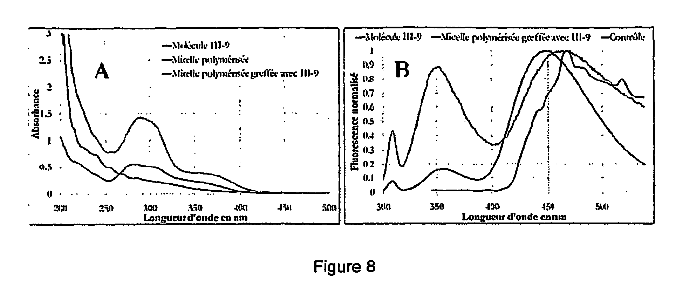

Les spectres d'absorption de la Figure 8 montrent que le dérivé aminé de l'acide folique III-9 est bien présent à la surface des micelles polymérisées du fait de la présence du pic à 285nm. Les spectres de fluorescence des micelles polymérisées fonctionnalisées montrent une évolution des pics de fluorescence du dérivé aminé de l'acide folique à 360nm et 450nm.

### 5.3 Fonctionnalisation par le PEG

La potentielle phagocytose des micelles de taille manométrique selon l'invention par les macrophages peut être évitée en greffant sur ces micelles des chaînes de PEG ou PolyEthylèneGlycol. La présence de PEG à la surface des micelles crée une couche externe inerte empêchant l'adhésion des opsonines, rendant donc lesdites micelles furtives vis-à-vis des macrophages et augmentant ainsi leur temps de circulation dans le compartiment sanguin.

Le couplage d'un méthoxyPEG-amine 5000 à la surface de la micelle polymérisée s'effectue à pH 12 avec de la DCC comme agent de couplage. Ce greffage est réalisé en fort excès de dérivé PEG (25 équivalents) et d'agent de couplage (100 équivalents). Les micelles dérivatisées sont purifiées par une filtration suivie d'une colonne par exclusion de taille.

### EXEMPLE 6 : ETUDE TOXICOLOGIQUE DES MICELLES POLYMERISEES

La distribution des micelles polymérisées dans l'organisme est un paramètre primordial à tester afin de déterminer le mode d'excrétion et le mode d'accumulation desdites micelles polymérisées.

Pour réaliser cette étude, l'amphiphile II-4 est radiomarqué au carbone 14 afin de synthétiser la micelle polymérisée radiomarquée pour une étude de pistage par autoradiographie chez le rat. L'amphiphile radiomarqué au carbone 14 est obtenu avec une activité spécifique de 7.6µCi/mg. L'administration des micelles polymérisées chez le rat s'effectue à une dose de 4MBq/kg et 100mg/kg pour un volume d'administration de2.5ml/kg. Pour ce faire, une solution à 40mg/ml est nécessaire avec une activité spécifique de 7.6µCi/mg.

L'étude d'accumulation/élimination des micelles polymérisées radiomarquées au carbone 14 a été menée sur 3 rats males de type Wistar. Une dose unique de 100mg/kg des micelles polymérisées [¹⁴C] a été administrée par voie intraveineuse (bolus) à chacun des rats, et les animaux ont été euthanasiés à 10 minutes, 24 heures et 48 heures. Les urines des rats 24 heures et 48 heures ont été récoltées afin de mesurer leur activité par scintillation liquide. La quantification des niveaux de radioactivité dans les tissus a été effectuée par radioluminographie de sections spécifiques des rats, obtenues par cryomicrotomie.

L'analyse des radioluminogrammes du rat 10 minutes, i.e. euthanasié à 10 minutes, met en évidence une distribution généralisée de radioactivité dans le corps du rat due à une concentration élevée de micelles polymérisées dans le sang. Des niveaux élevés de radioactivité ont été détectés dans certains tissus tels que les poumons, les reins et ses substances médullaires, le foie, les glandes adrénales ou la rate.

L'analyse des radioluminogrammes des rats 24 heures et 48 heures, i.e. respectivement euthanasié à 24 et 48 heures, révèle une concentration de radioactivité élevée dans le foie, la paroi intestinale, la rate, les glandes adrénales, les reins et la moelle osseuse.

## Revendications

1. Micelles polymérisées **caractérisées en ce qu'**elles comprennent des molécules amphiphiles polymérisées obtenues à partir de molécules amphiphiles
de formule générale A-X-B-L-Z
où A représente 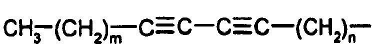 ou CH₂=CH- ou CH₂=CH-C₆H₄- , n et m, identiques ou différents, étant des entiers de 1 à 16;
où X représente CO-NH ou NH-CO ou une liaison, X est une liaison si B est une liaison et L est une liaison ;
où B représente 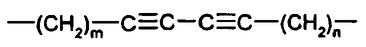 ou une liaison, n et m, identiques ou différents, étant des entiers de 1 à 16 ;
où L représente -(CH₂)ᵣ-CH[NH-CO-A']- ou une liaison, r étant un entier de 1 à 16 et A' représente A ;
où Z représente 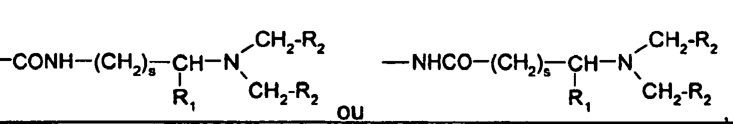 s étant un entier de 1 à 16 avec R₂ représentant COOH ou SO₃H ou OSO₃H ou OPO₃H₂ ou OPO₂H₂ avec R₁ représentant H ou un radical COOH ou SO₃H ou OSO₃H ou OPO₃H₂ ou OPO₂H₂ ou un groupe -CO-NH-(CH₂)ₜ-CH₃, t étant un entier de 1 à 16, ou Z peut être également des têtes polaires hydrophiles neutres, de type sucre ou Polysaccharide, ainsi que leurs sels d'addition avec un acide ou une base pharmaceutiquement acceptable.

2. Micelles polymérisées selon la revendications 1, **caractérisées en ce qu'**elles comprennent des molécules amphiphiles polymérisées obtenues à partir de molécules amphiphiles de formule générale
A-X-B-L-Z
où A représente 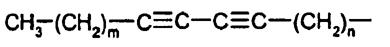 n et m, identiques ou différents, étant des entiers de 1 à 16;
où X représente CO-NH ou NH-CO ou une liaison, X est une liaison si B est une liaison et L est une liaison ;
où B représente 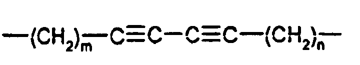 ou une liaison, n et m, identiques ou différents, étant des entiers de 1 à 16 ;
où L représente -(CH₂)ᵣ-CH[NH-CO-A']- ou une liaison, r étant un entier de 1 à 16 et A' représente A ou CH₂=CH- ou CH₂=CH-C₆H₄- ;
où Z représente 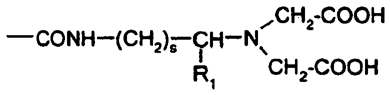 ou 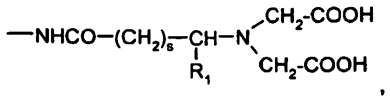 s étant un entier de 1 à 16 avec R₁ représentant H ou un radical COOH ou un groupe -CO-NH-(CH₂)ₜ-CH₃, t étant un entier de 1 à 16, ou Z peut être également des têtes polaires hydrophiles, neutres, de type sucre ou polysaccharide, ainsi que leurs sels d'addition avec un acide ou une base pharmaceutiquement acceptable.

3. Micelles polymérisées selon l'une des revendications 1 à 2, **caractérisées en ce que** la tête polaire Z est fonctionnalisée.

4. Micelles polymérisées selon la revendication 3 4, caractérisées en ce la tête polaire Z est fonctionnalisée par un acide folique.

5. Micelles polymérisées selon l'une quelconque des revendications 1 à 4, **caractérisées en ce qu'**elles incluent un ou des composés hydrophobes.

6. Micelles polymérisées selon l'une quelconque des revendications 1 à 5 6, **caractérisées en ce que** les molécules amphiphiles polymérisées sont obtenues à partir de
la molécule amphiphile II-4 de formule

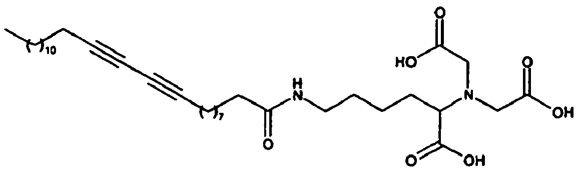

7. Micelles polymérisées selon l'une quelconque des revendications 1 à 5 6, **caractérisées en ce que** les molécules amphiphiles polymérisées sont obtenues à partir de la molécule amphiphile II-23 de formule

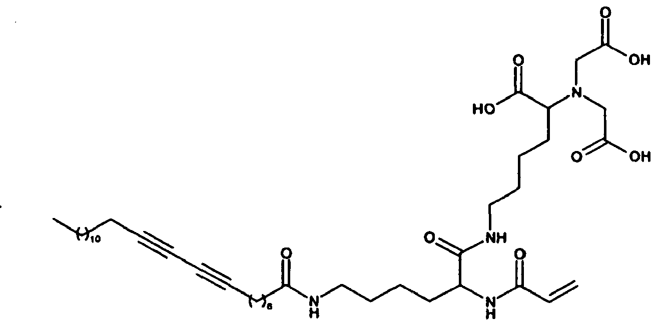

8. Micelles polymérisées selon l'une quelconque des revendications 1 à 5, **caractérisées en ce que** les molécules amphiphiles polymérisées sont obtenues à partir de la molécule amphiphile de formule

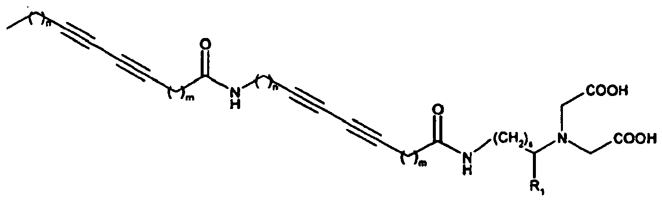

9. Micelles polymérisées selon l'une quelconque des revendications 1 à 5 6, **caractérisées en ce que** les molécules amphiphiles polymérisées sont obtenues à partir de la molécule amphiphile de formule

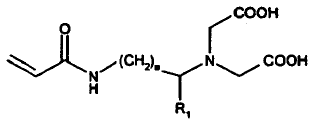

10. Micelles polymérisées selon l'une quelconque des revendications 1 à 5, **caractérisées en ce que** les molécules amphiphiles polymérisées sont obtenues à partir de la molécule amphiphile de formule

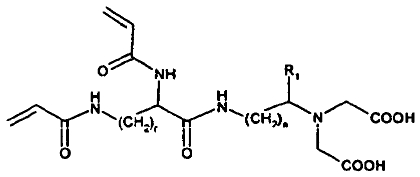

11. Micelles polymérisées selon l'une quelconque des revendications 1 à 5 6, **caractérisées en ce que** les molécules amphiphiles polymérisées sont obtenues à partir de la molécule amphiphile de formule

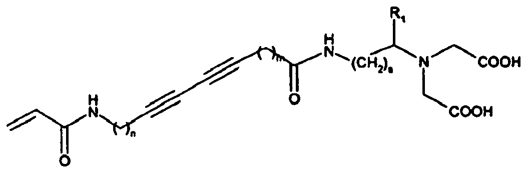

12. Micelles polymérisées selon l'une quelconque des revendications 1 à 5 6, **caractérisées en ce que** les molécules amphiphiles polymérisées sont obtenues à partir de la molécule amphiphile de formule

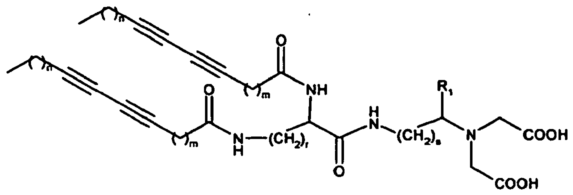

13. Composition **caractérisée en ce qu'**elle comprend des micelles polymérisées selon l'une quelconque des revendications 1 à 12.

14. Composition selon la revendication 13, **caractérisée en ce qu'**elle comprend un ou plusieurs excipients pharmaceutiquement acceptables.

15. Utilisation des micelles polymérisées selon l'une quelconque des revendications 1 à 12 comme vecteurs de molécules hydrophobes.

16. Utilisation des micelles polymérisées selon la revendication 15, **caractérisées en ce que** les molécules hydrophobes sont des principes actifs hydrophobes.

17. Procédé d'obtention des micelles polymérisées selon l'une quelconque des revendications 1 à 12, **caractérisé en ce qu'**il comprend les étapes suivantes :
- les molécules amphiphiles à polymériser selon les revendications 1 à 12 sont auto-assemblées en micelles sphériques ;
- les micelles sphériques auto-assemblées sont polymérisées.

18. Procédé selon la revendication 17, **caractérisé en ce que** les micelles sphériques auto-assemblées sont photopolymérisées.

19. Procédé selon la revendication 17, **caractérisé en ce que** les micelles sphériques auto-assemblées sont polymérisées par polymérisation radicalaire.

## Claims

1. Polymerised micelles **characterised in that** they comprise polymerised amphiphilic molecules obtained starting from amphiphilic molecules of general formula A-X-B-L-Z
wherein A represents 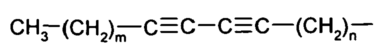 or CH₂=CH- or CH₂=CH-C₆H₄- , n and m, which may be the same or different, being integers from 1 to 16;
wherein X represents CO-NH or NH-CO or a bond, X is a bond if B is a bond and L is a bond;
wherein B represents 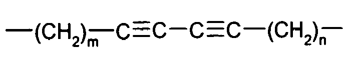 or -CH=CH-C₆H₄- or a bond, n and m, which may be the same or different, being integers from 1 to 16;
wherein L represents -(CH₂)ᵣ-CH[NH-CO-A']- or a bond, r being an integer from 1 to 16, and A' represents A;
wherein Z represents 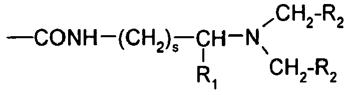 or 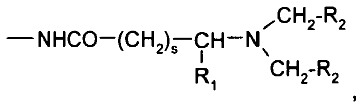 s being an integer from 1 to 16, with R₂ representing COOH or SO₃H or OSO₃H or OPO₃H₂ or OPO₂H₂, with R₁ representing H or a radical COOH or SO₃H or OSO₃H or OPO₃H₂ or OPO₂H₂ or a group -CO-NH-(CH₂)ₜ-CH₃, t being an integer from 1 to 16, or Z may also be a neutral hydrophilic polar head of the sugar or polysaccharide type, and also addition salts thereof with a pharmaceutically acceptable acid or base.

2. Polymerised micelles according to claim 1, **characterised in that** they comprise polymerised amphiphilic molecules obtained starting from amphiphilic molecules of general formula
A-X-B-L-Z
wherein A represents 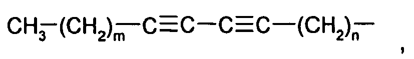 n and m, which may be the same or different, being integers from 1 to 16;
wherein X represents CO-NH or NH-CO or a bond, X is a bond if B is a bond and L is a bond;
wherein B represents 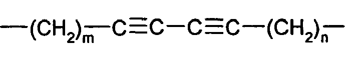 or a bond, n and m, which may be the same or different, being integers from 1 to 16;
wherein L represents -(CH₂)ᵣ-CH[NH-CO-A']- or a bond, r being an integer from 1 to 16, and A' represents A or CH₂=CH- or CH₂=CH-CeH₄-;
wherein Z represents 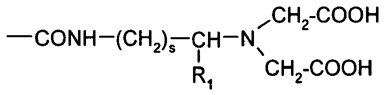 or 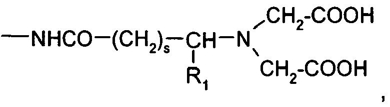 s being an integer from 1 to 16, with R₁ representing H or a radical COOH or a group -CO-NH-(CH₂)ₜ-CH₃, t being an integer from 1 to 16, or Z may also be a neutral hydrophilic polar head of the sugar or polysaccharide type, and also addition salts thereof with a pharmaceutically acceptable acid or base.

3. Polymerised micelles according to one of claims 1 to 2, **characterised in that** the polar head Z is functionalised.

4. Polymerised micelles according to claim 3, **characterised in that** the polar head Z is functionalised by a folic acid.

5. Polymerised micelles according to any one of claims 1 to 4, **characterised in that** they include one or more hydrophobic compounds.

6. Polymerised micelles according to any one of claims 1 to 5, **characterised in that** the polymerised amphiphilic molecules are obtained starting from the amphiphilic molecule II-4 of formula

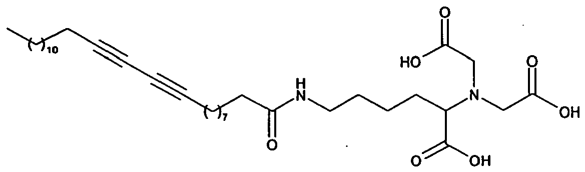

7. Polymerised micelles according to any one of claims 1 to 5, **characterised in that** the polymerised amphiphilic molecules are obtained starting from the amphiphilic molecule II-23 of formula

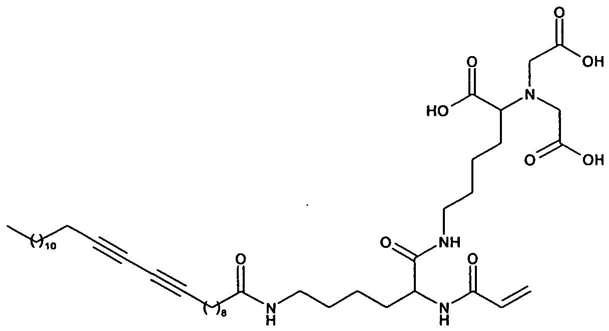

8. Polymerised micelles according to any one of claims 1 to 5, **characterised in that** the polymerised amphiphilic molecules are obtained starting from the amphiphilic molecule of formula

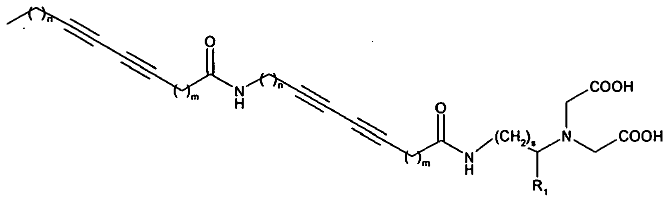

9. Polymerised micelles according to any one of claims 1 to 5, **characterised in that** the polymerised amphiphilic molecules are obtained starting from the amphiphilic molecule of formula

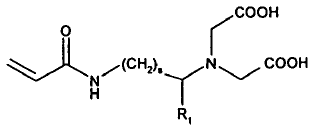

10. Polymerised micelles according to any one of claims 1 to 5, **characterised in that** the polymerised amphiphilic molecules are obtained starting from the amphiphilic molecule of formula

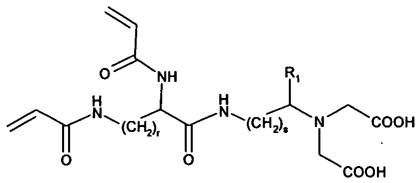

11. Polymerised micelles according to any one of claims 1 to 5, **characterised in that** the polymerised amphiphilic molecules are obtained starting from the amphiphilic molecule of formula

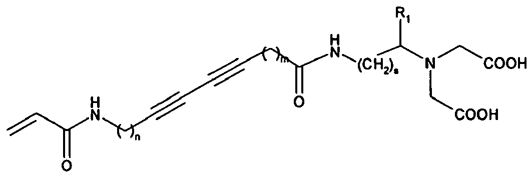

12. Polymerised micelles according to any one of claims 1 to 5, **characterised in that** the polymerised amphiphilic molecules are obtained starting from the amphiphilic molecule of formula

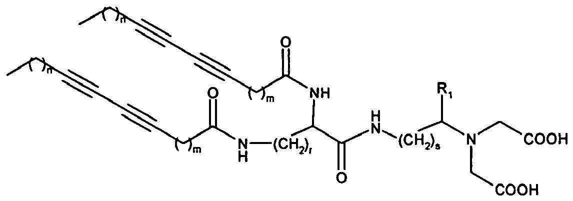

13. Composition **characterised in that** it comprises polymerised micelles according to any one of claims 1 to 12.

14. Composition according to claim 13, **characterised in that** it comprises one or more pharmaceutically acceptable excipients.

15. Use of polymerised micelles according to any one of claims 1 to 12 as vectors of hydrophobic molecules.

16. Use of polymerised micelles, according to claim 15, **characterised in that** the hydrophobic molecules are hydrophobic active ingredients.

17. Process for obtaining polymerised micelles according to any one of claims 1 to 12, **characterised in that** it comprises the following steps:
- the amphiphilic compounds to be polymerised according to claims 1 to 12 are self-assembled into spherical micelles;
- the self-assembled spherical micelles are polymerised.

18. Process according to claim 17, **characterised in that** the self-assembled spherical micelles are photopolymerised.

19. Process according to claim 17, **characterised in that** the self-assembled spherical micelles are polymerised by free radical polymerisation.

## Patentansprüche

1. Polymerisierte Mizellen, **dadurch gekennzeichnet, dass** sie polymerisierte amphiphile Moleküle umfassen, welche erhalten worden sind ausgehend von amphiphilen Molekülen der allgemeinen Formel A-X-B-L-Z,
worin A CH₃-(CH₂)ₘ-C≡C-C≡C-(CH₂)ₙ- oder CH₂=CH- oder CH₂=CH-C₆H₄- bedeutet und n und m, die identisch oder verschieden sind, ganze Zahlen mit Werten von 1 bis 16 bedeuten;
worin X CO-NH oder NH-CO oder eine Bindung darstellt, wobei X eine Bindung bedeutet, wenn B eine Bindung und L eine Bindung darstellen;
worin B -(CH₂)ₘ-C≡C-C≡C-(CH₂)ₙ- oder -CH=CH-C₆H₄- oder eine Bindung bedeutet und n und m, die identisch oder verschieden sind, ganze Zahlen mit Werten von 1 bis 16 bedeuten;
worin L -(CH₂)ᵣ-CH[NH-CO-A']- oder eine Bindung bedeutet, r eine ganze Zahl mit einem Wert von 1 bis 16 bedeutet und A' A bedeutet;
worin Z 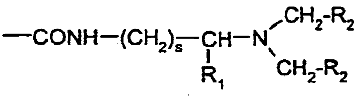 oder 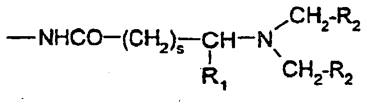 bedeutet, s eine ganze Zahl mit einem Wert von 1 bis 16 darstellt, R₂ COOH oder SO₃H oder OSO₃H oder OPO₃H₂ oder OPO₂H₂ darstellt, R₁ H oder einen Rest COOH oder SO₃H oder OSO₃H oder OPO₃H₂ oder OPO₂H₂ oder eine Gruppe -CO-NH-(CH₂)t-CH3 bedeutet, t eine ganze Zahl mit einem Wert von 1 bis 16 bedeutet, oder Z auch neutrale hydrophile polare Enden vom Zucker- oder Polysaccharid-Typ bedeuten kann, sowie deren Additionssalze mit einer pharmazeutisch annehmbaren Säure oder
Base.

2. Polymerisierte Mizellen nach Anspruch 1, **dadurch gekennzeichnet, dass** sie polymerisierte amphiphile Moleküle enthalten, die erhalten worden sind ausgehend von amphiphilen Molekülen der allgemeinen Formel
A-X-B-L-Z
worin A CH₃-(CH₂)ₘ-C≡C-C≡C-(CH₂)ₙ- bedeutet, n und m, die identisch oder verschieden sind, ganze Zahlen mit Werten von 1 bis 16 bedeuten;
worin X CO-NH oder NH-CO oder eine Bindung bedeutet, wobei X eine Bindung darstellt, wenn B eine Bindung bedeutet und L eine Bindung bedeutet;
worin B -(CH₂)ₘ-C≡C-C≡C-(CH₂)ₙ- oder eine Bindung bedeutet, n und m, die identisch oder verschieden sind, ganze Zahlen mit Werten von 1 bis 16 bedeuten;
worin L -(CH₂)ᵣ-CH[NH-CO-A']- oder eine Bindung bedeutet, r eine ganze Zahl mit einem Wert von 1 bis 16 darstellt und A' A oder CH₂=CH- oder CH₂=CH-C₆H₄- bedeutet;
worin Z 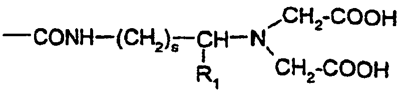 oder 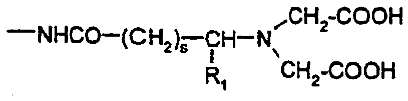 bedeutet, s eine ganze Zahl mit einem Wert von 1 bis 16 darstellt, wobei R₁ H oder einen Rest COOH oder eine Gruppe -CO-NH-(CH₂)ₜ-CH₃ bedeutet, t eine ganze Zahl mit einem Wert von 1 bis 16 bedeutet, oder Z auch neutrale hydrophile polare Enden vom Zucker- oder Polysaccharid-Typ bedeuten kann, sowie deren Additionssalze mit einer pharmazeutisch annehmbaren Säure oder Base.

3. Polymerisierte Mizellen nach einem der Ansprüche 1 bis 2, **dadurch gekennzeichnet, dass** das polare Ende Z funktionalisiert ist.

4. Polymerisierte Mizellen nach Anspruch 3, **dadurch gekennzeichnet, dass** das polare Ende Z durch eine Folsäure funktionalisiert ist.

5. Polymerisierte Mizellen nach einem der Ansprüche 1 bis 4, **dadurch gekennzeichnet, dass** sie eine oder mehrere hydrophobe Verbindungen einschließen.

6. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül II-4 der Formel

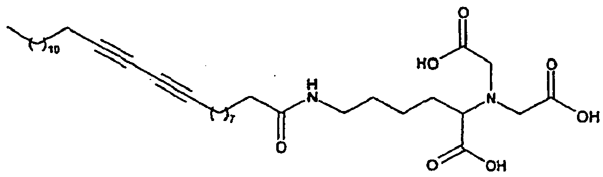

7. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül II-23 der Formel

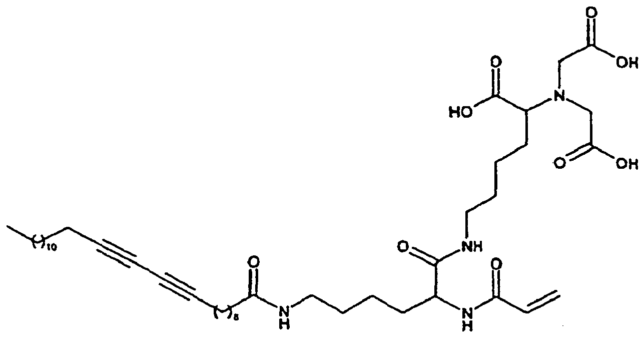

8. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül der Formel

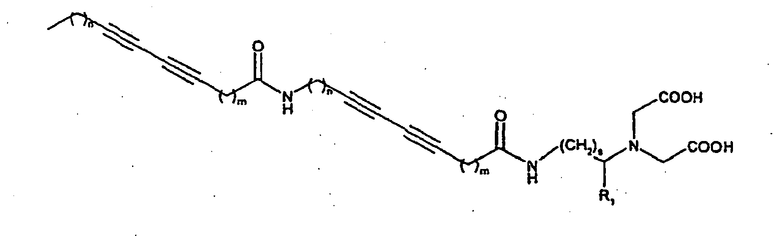

9. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül der Formel

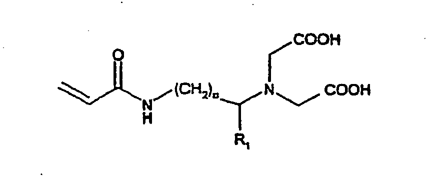

10. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül der Formel

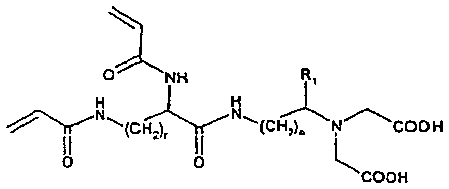

11. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül der Formel

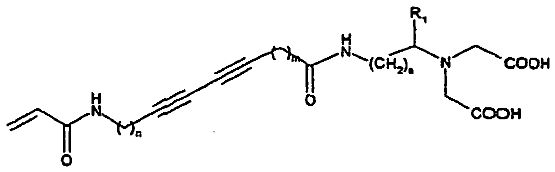

12. Polymerisierte Mizellen nach einem der Ansprüche 1 bis 5, **dadurch gekennzeichnet, dass** die polymerisierten amphiphilen Moleküle erhalten worden sind ausgehend von dem amphiphilen Molekül der Formel

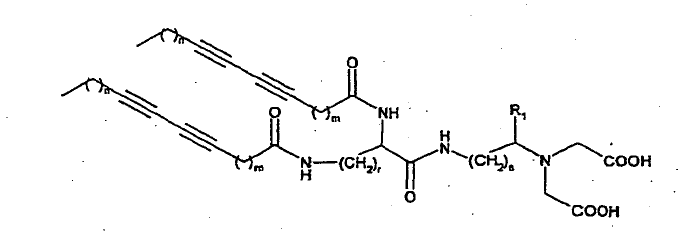

13. Zusammensetzung, **dadurch gekennzeichnet, dass** sie polymerisierte Mizellen nach einem der Ansprüche 1 bis 12 umfasst.

14. Zusammensetzung nach Anspruch 13, **dadurch gekennzeichnet, dass** sie einen oder mehrere pharmazeutisch annehmbare Hilfsstoffe enthält.

15. Verwendung der polymerisierten Mizellen nach einem der Ansprüche 1 bis 12 als Vektoren für hydrophobe Moleküle.

16. Verwendung der polymerisierten Mizellen nach Anspruch 15, **dadurch gekennzeichnet, dass** die hydrophoben Moleküle hydrophobe Wirkstoffe sind.

17. Verfahren zur Herstellung der polymerisierten Mizellen nach einem der Ansprüche 1 bis 12, **dadurch gekennzeichnet, dass** es die folgenden Stufen umfasst:
- die gemäß den Ansprüchen 1 bis 12 zu polymerisierenden amphiphilen Moleküle werden zu sphärischen Mizellen selbst-assembliert;
- die selbst-assemblierten sphärischen Mizellen werden polymerisiert.

18. Verfahren nach Anspruch 17, **dadurch gekennzeichnet, dass** die selbst-assemblierten sphärischen Mizellen photopolymerisiert werden.

19. Verfahren nach Anspruch 17, **dadurch gekennzeichnet, dass** die selbst-assemblierten sphärischen Mizellen durch radikalische Polymerisation polymerisiert werden.
